# EUROPEAN PATENT APPLICATION

(11) **EP 1 285 985 A1**
(43) Date of publication of application: **26.02.2003**
(21) Application number: 02018521.1
(22) Date of filing: 16.08.2002
(51) Int. Cl.: D04H 1/64, A47K 10/16, A61K 7/48, A47L 13/17

(54) **Disintegratable pre-moistened wipes substantially free of boric acid and its derivatives and lotion therefor**

(30) Priority: 22.08.2001 US 934867
(71) Applicant: AIR PRODUCTS POLYMERS, L.P., Allentown, PA 18195-1501 (US)
(72) Inventor: Goldstein, Joel Erwin, Allentown, PA 18104-2903 (US); Welty, Jeffrey Allen, Hellertown, PA 18055 (US)
(74) Representative: Kador & Partner

(57) **Abstract**

A packaged pre-moistened wet wipe comprising a web of nonwoven fibers bonded with a water soluble and/or dispersible polymeric binder in contact with an aqueous composition which is substantially free of boric acid and contains a sufficient amount of a water-binding compound, which preferably is a salt of an organic or inorganic acid, to prevent the dissolution/redispersion of the water soluble/dispersible components of the binder composition while imbuing the nonwoven with a wet tensile strength that is at least as great as that afforded by a 4 wt% boric acid solution.

## Description

### FIELD OF THE INVENTION

This invention relates to a pre-moistened nonwoven towelette, or wet wipe, that is readily water disposable and/or water dispersible.

### BACKGROUND OF THE INVENTION

The issue of disposability of products is of great concern to the nonwovens industry. The industry wishes to offer pre-moistened toilet tissue on a roll and other pre-moistened wipes that will be truly flushable. In another words, the webs must disintegrate in the toilet water under gentle agitation without the addition of temperature or chemicals. Current products either are dry or do not possess sufficient strength when wet. Other products are not truly flushable in that the web does not disintegrate upon being flushed in a toilet.

Wet-packaged skin cleansing and refreshing tissues are well-known commercially, generally referred to as towelettes, wet wipes, fem wipes and the like. These may comprise an absorbent sheet made of paper, prepared or treated to impart wet strength thereto, having the dimensions of the usual washcloth and packaged wet in folded condition, individually in impervious envelopes or in multiples in closed containers. The liquid employed in the pre-moistening of the bonded nonwoven web is generally an aqueous composition, or lotion, which may further contain a surface active agent and a humectant and, in some instances, also a scenting agent. Instead of individual packaging of such moist sheets, they are often marketed in reclosable containers having any desired convenient number of such folded sheets.

Polymeric binders used in making the nonwoven substrates generally comprise polyvinyl alcohol (PVOH) and/or PVOH-stabilized vinyl acetate (VAc) or vinyl acetateethylene (VAE) emulsion polymers with boric acid or its derivatives being added to the aqueous lotion composition in an attempt to maintain the integrity of the wet nonwoven substrate. Sometimes the boric acid is added along with alkali metal bicarbonates. However, high levels of the alkali metal bicarbonates are necessary as a lotion slowly causes the web to lose performance. Either the aqueous lotion slowly dissolves the PVOH allowing this protective colloid to redisperse any other polymer binder, or the bicarbonate decomposes slowly to carbon dioxide which then evaporates out of the solution, even through the plastic typically used in the construction of the containers. In addition, the use of boric acid and its derivatives for preventing the lotion from causing disintegration of the web has fallen into disfavor due to some perception as to its potential harmful effects near mucous membranes.

US 4,245,744 discloses nonwoven fiber sheets impregnated with PVOH-containing vinyl acetate-based polymers in which the nonwoven sheets are maintained in contact with a dilute aqueous solution of a precipitating or gelling agent for PVOH, such as boric acid.

US 4,372,447 discloses nonwoven sheets bonded with polyvinyl alcohol. The sheets being maintained in contact with a dilute aqueous solution of boric acid or a non-alkaline aqueous solution of a salt which acts as a precipitating or gelling agent for PVOH.

US 5,252,332 discloses a packaged towelette comprising a sheet of nonwoven fibers impregnated with a PVOH-containing binder and in contact with an aqueous solution containing boric ions and bicarbonate ions.

US 5,629,081 discloses a pre-moistened, dispersible and biodegradable wet wipe comprising a web of nonwoven fibers contacted with a PVOH-containing binder. The binder-contacted web further comprises an aqueous lotion solution comprising 0.1-0.9 wt% boric acid and 5-8 wt% alkali metal bicarbonate, based on weight of the lotion.

More recent technology for disposable wet wipes is based upon a highly carboxylated polymer emulsion. The backbone of this polymer system can be either vinyl acetate, acrylic esters or mixtures thereof. Styrene, styrene-butadienes and styrene-acrylics may also compose the backbone. The technology entails having the carboxylate converted to a salt of a bivalent or trivalent atom such as calcium or magnesium. This makes the oligomeric chain containing the carboxylic acid insoluble in water. When the wipe hits the toilet water, the calcium is exchanged for a monovalent cation, such as sodium. The sodium salt of the carboxylate is more water soluble, therefore, the polymer can dissolve in the toilet water so the web will redisperse. The problem with this technique is it requires the use of divalent cation traps. Since many toilet bowls contain hard water, the concentration of such salts in the hard water could retard, inhibit and/or prevent the rapid disintegration of the web.

US 5,384,189 discloses a water decomposable nonwoven fatric in which the fibers are bonded to one another with a water-soluble binder comprising an unsaturated carboxylic acid/unsaturated carboxylic acid ester copolymer in which 1-60 mole% of the repeating units derived from the carboxylic acid is in the form of a salt and which is soluble in tap water but is insoluble in an aqueous solution containing not less than 0.5 wt% of a neutral inorganic salt comprising a monovalent ion.

US 5,935,880 discloses a dispersible nonwoven fabric which in the case of a wet wipe is in conjunction with a solution containing about 100 ppm of calcium ions.

US 5,972,805 discloses a water-soluble polymeric binder composition for use in making nonwoven webs comprising 25-90 wt% unsaturated carboxylic acid/unsaturated carboxylic acid esters/ester copolymer, 10-75 wt% divalent ion inhibitor and 0-10 wt% plasticizer. The water-soluble binder composition is soluble in an aqueous environment having a divalent ion concentration listing about 50 ppm and a monovalent concentration of less than about 0.5 wt%.

Other relevant art includes:
US 5,256,417 which discloses a packaged towelette comprising a sheet of nonwoven fibers impregnated with a binder which is a PVOH or an aqueous polymer emulsion containing PVOH as a protective colloid, the sheet being maintained in a wet condition within the package by contact with a non-aqueous lotion composition which is a liquid organic compound that is a non-solvent for PVOH, and
US 5,667,635 which discloses a pre-moistened, wet wipe consisting of three uncreped through air-dried tissue plies that are attached to each other by edge embossing.

### SUMMARY OF THE INVENTION

The present invention provides a pre-moistened wet wipe comprising a nonwoven web, or substrate, of fibers bonded with a water soluble or redispersible polymeric binder composition and in contact with an aqueous lotion composition that will not dissolve or redisperse the binder off the nonwoven web, which lotion composition is also substantially free of boric acid and its derivatives. The binder may comprise any water soluble and/or water dispersible polymer capable of binding together the fibers composing the nonwoven web but preferably is either a polyvinyl alcohol (PVOH), an aqueous PVOH-stabilized polymer emulsion, a blend of a PVOH and an aqueous polymer emulsion or a combination thereof. The aqueous lotion composition contains one or more compounds which preferentially tie up, or bind, the water in the aqueous lotion, i.e., water-binding compounds, so that the water does not substantially dissolve any water soluble fraction of the nonwoven binder which in turn would cause redispersal of any other portion of the nonwoven binder resulting in disintegration of the web. The water-binding compound is present in sufficient quantity so as to bind enough of the water in the lotion composition to afford a nonwoven substrate having a wet tensile strength at least as great as that achieved by the same bonded nonwoven substrate in contact with an aqueous 4 wt% boric acid solution. The add-on amounts of the lotion composition and the boric acid solution are irrelevant in the wet tensile strength comparison.

Another embodiment of the invention is an aqueous lotion composition for nonwoven wet wipes that will not dissolve and/or redisperse the water soluble/redispersible binder from the web while also being substantially free of boric acid and its derivatives.

The present invention provides a pre-moistened wipe having the following advantages:
- long shelf life,
- good tensile strength in the aqueous lotion,
- good dry tensile strength required for manufacturing,
- freedom from having to use any boric acid or its derivatives in the aqueous lotion, and
- rapid disintegration of the disposed, used wipe under the ambient conditions of typical water-containing toilets.

Another advantage is that a plasticizer, such as a polyethylene glycol, as typically used in aqueous lotions, may not be necessary due to the good hand feel that the binders have when pre-moistened with the aqueous lotion of the invention.

Another advantage is that the degree of hardness of the toilet water will not adversely affect the disintegration of the disposable wet wipe.

### DETAILED DESCRIPTION OF THE INVENTION

The present invention is directed to a nonwoven wet wipe that is both safe to use and also flushes and disperses and finally biodegrades in appropriate environments. The term "wet wipe" for purposes of the present invention means a pre-moistened nonwoven web of fibers that can be used, for example, for cleansing purposes and includes items such as towelettes, wipes, e.g., baby wipes, hemorrhoid wipes, feminine hygiene wipes, bedridden patient wipes, bathroom cleaning wipes and the like, and pre-moistened toilet paper. The term "dispersible" means the ability of the nonwoven wet wipe to disintegrate in water or other aqueous medium. As the context permits in terms of the nonwoven binder compositions, use of the terms "solubilize", "soluble", "dissolve", "dissolution" and like terms also contemplate the terms "(re)disperse", "(re)dispersion" and "(re)dispersible".

The nonwoven wet wipe of the invention comprises a nonwoven fibrous material bonded with a water soluble binder and/or water dispersible binder ("water soluble/dispersible") in contact with an aqueous lotion composition containing a water-binding compound, i.e., a compound that is preferentially dissolved in water relative to any water soluble/dispersible portion of the nonwoven binder composition. The water-binding compound is present in the aqueous lotion in at least sufficient quantity to prevent the dissolution of such water soluble/dispersible portion of the nonwoven binder to such an extent as to afford a nonwoven having a wet tensile strength that is equal to or greater than that of the same bonded nonwoven in contact with an aqueous 4 wt% boric acid solution.

In general, the wet wipe comprises primarily cellulose wood pulp fibers, typically with an added amount of textile fibers to enhance wet and dry strength. Typically, the wood pulp fibers comprise about 75-90 wt% and the textile fibers about 5-25 wt% of the nonwoven substrate. The preferred textile fibers include rayon, cotton, wool, acetate, or tencel fibers.

Wood pulp (alone or blended with natural or synthetic fibers) can be processed by dry (air-laid, carded, rando) or wet-laid processes. Nonwoven webs produced by air-laid processes are preferred due to minimal hydrogen bonding of fibers in the finished product compared to wet-laid nonwovens. Air-laid processes impart little or no inherent integrity to the web which must be overcome with agitation to achieve complete disintegration of the web.

The initial treatment to coat or impregnate the nonwoven web with the water soluble/dispersible binder may be carried out (1) by immersing the webs, or running lengths of the web in an aqueous composition of the water soluble/dispersible binder, especially a PVOH or an aqueous polymer emulsion either stabilized with PVOH as the protective colloid or containing post-added PVOH, or (2) by applying such water soluble/dispersible binder to the surfaces of the nonwoven web of fibers by spraying, by padding, by roller, or other types of applicator. Following drying, the treated nonwoven web may be then cut to desired sized sheets for the intended use. Coarse, individual sheets precut to desired size may be treated with the aqueous PVOH or polymer emulsions and then dried.

Exemplary of suitable water soluble/dispersible binders are PVOHs, aqueous polymer emulsions either stabilized with PVOH as the protective colloid or containing post-added PVOH, polyethylene glycols, polyacrylamides and polyvinyl pyrrolidones.

The preferred water soluble/dispersible nonwoven binders for use include 75-90 mole% hydrolyzed, preferable 86-89 mole% hydrolyzed PVOHs alone or blended with polymer emulsions. It is preferred to use PVOHs having a high molecular weight (DPn > 600 and ranging up to 2500 and more). Any polymer emulsion known in the art as a binder for nonwovens can be used when blended with the PVOH. It is also preferred that the emulsion polymer be non-crosslinking, e.g., does not contain polymerized N-methylolacrylamide, and most desirably contains PVOH as the protective colloid or stabilizing system, in its preparation by aqueous emulsion polymerization. PVOH stabilized vinyl acetate (VAc) or vinyl acetate/ethylene (VAE) polymer emulsions are preferred due to their ease of water dispersibility.

The ratio of PVOH to emulsion solids will depend upon the type of product being made and the choice of the PVOH and polymer emulsion. The preferred range is a minimum of 5 parts PVOH (dry) to 100 parts emulsion solids up to and including 100% PVOH (i.e., no emulsion polymer binder). The VAEs tend to be more hydrophobic and better film formers than the VAc polymers and require, accordingly, higher amounts of PVOH, i.e., up to 200 parts of PVOH per 100 parts of emulsion solids compared to VAc polymers which may require up to 100 parts PVOH per 100 parts emulsion solids.

The amount of polymer binder calculated on the dry basis, applied to the fibrous starting web, is that amount which is at least sufficient to bind fibers together to form a self-sustaining web and suitably ranges from about 3-100% or more by weight of the starting web. Where PVOH is the polymer binder, about 3-20 wt% preferably is applied to the web. Where an aqueous polymer emulsion containing PVOH is the binder, about 5-50 wt% preferably is applied, the emulsion containing 20-200 parts PVOH per 100 parts emulsion on a dry basis.

The impregnated web is then dried by passing it through an air oven for sufficient times and temperatures, such as drying at 150-200°F (66-93°C for 4-6 minutes in lab tests designed to simulate production conditions).

The pre-moistened wipes are packaged in contact with an aqueous lotion composition containing in water a water-binding compound to temporarily insolubilize the water soluble/dispersible binder. In addition, the lotion composition may also comprise alcohols, preservatives, cleansing agents, fragrances, moisturizers, humectants, surfactants and softeners as is well known and practiced in the art.

The amount of the aqueous lotion composition applied to, or in contact with, the nonwoven may range from 150-1000 wt% of the web, preferably 200-400 wt%. The aqueous lotion may comprise 40-99 wt% water, preferably 75-97 wt%, and most desirably 90-95 wt% water.

The aqueous lotion composition should be substantially free of boric acid or its derivatives such as borate salts, i.e., less than 0.05 wt%, preferably less than 0.01 wt%, and most preferably no detectable amounts of those materials.

The water-binding compound suitable for use in the aqueous lotion composition is any compound which, when added in sufficient quantity, prevents the dissolution of the water-soluble component or the redispersion of the water-dispersible component of the nonwoven binder. The minimum amount of this water-binding compound is that amount which prevents enough of the water soluble/dispersible portion of the binder from dissolving and/or redispersing so as to reduce the wet strength of the pre-moistened wipe in contact with the lotion composition to less than that of the same nonwoven web in contact with the same add-on amount of a 4 wt% solution of boric acid.

The amount of the water-binding materials can range from 3 to about 100 wt%, preferably 5-95 wt%, and most preferably 20-90 wt%, of the lotion "solids", i.e., the components other than water comprising the aqueous lotion composition. The usage range depends on the material itself and its ability to bind up the water in the lotion. The lotion composition does not need to be saturated with the water-binding material but it does need to contain enough of it such that an insufficient amount of water is free to dissolve the water soluble portion of the nonwoven binder.

Water binding compounds include ammonium, mono- and polyvalent metal salts of inorganic and organic acids, such as the alkali and alkaline earth metal salts. Suitable metals include the alkali metals lithium, sodium, potassium and the alkaline earth metals magnesium and calcium. Suitable acid moieties include organic acids such as carbonic acid, citric acid, acetic acid and succinic acid and inorganic acids such as phosphoric acid, sulfuric acid and hydrochloric acid.

Preferred materials for tying up the water comprise salts such as sodium chloride, sodium sulfate, ammonium acetate, sodium citrate, sodium carbonate, sodium bicarbonate, sodium lauryl sulfate, disodium laureth sulfosuccinate, sodium laureth sulfate, sodium nonylphenol ethoxylate sulfate, disodium phosphate, sodium carbonate and their potassium counterpart. The minimum amount of material for effectively tying up the water in an aqueous lotion composition varies with the particular material used and the other components present in the lotion composition.

The water-binding material may be a mixture of several components if desired, to moderate expense, bring some additional property to the lotion such as moisturizer, color stabilizer, or neutralizer and the like or for some other enhanced synergistic effect. If the level of the water-binding material is high enough such that little unbound water is available, the need for a biocide to protect the lotion may become unnecessary.

Some of the components presently used in aqueous lotions for pre-moistened wipes may also be suitable as water-binding compounds. Since these components are typically used in relatively small quantities in aqueous lotions, an appropriately increased amount must be added. Examples of such components are humectants and preservatives, such as disodium cocoamphodiacetate and potassium sorbate. Also, since such components are typically much more expensive than the water-binding salts, they may need to be used in combination with some quantity of a salt to achieve an economically acceptable water binding effect. In most cases the aqueous lotion compositions will be aqueous solutions containing the various components.

In the following examples, nonwoven fibrous webs of wood pulp were first bonded with VINAC® 911 emulsion (a blend of PVOH and a PVOH-stabilized VAc polymer emulsion available from Air Products Polymers, L.P.) by spraying the webs with a 10% solids binder composition to an add-on of 20 wt%. Next the dried, bonded nonwoven substrate was treated with 700 wt% of an aqueous solution containing the compounds in the amounts as set forth in Tables 1 and 2 by spraying the solution onto the web already placed in the jaws of an Instron tester for determining wet tensile values (g/5cm) at room temperature. The wet tensile strength of a nonwoven sprayed with a 700% loading of a 1% aqueous solution of dioctylsulfosuccinate sodium soda was g/5cm.

### Example 1

This example presents in Table 1 the wet tensile strength data (g/5cm) for flushable wipes prepared from a nonwoven substrate which comprised a 35 grams/m² web. In the runs designated as using Cottonelle and Tucks, the aqueous lotions from the commercial Cottonelle and Tucks wet wipes were obtained by squeezing the lotions out of the respective commercial products while using latex gloves and to these lotions the indicated amounts of sodium chloride were added prior to contacting the web. These lotions contained the additional components glycerin, witch hazel, propylene glycol, parabens, citric acid, sodium citrate and imidoazolidinyl urea .

**Table 1**

| **Flushable Wipes Solution** | **Avg. Peak Load (g/5cm)** |
|---|---|
| Dry | 239.8 |
| 4% Boric Acid | 35.4 |
| 25% Sodium Chloride | 53.6 |
| 5% NaCl/ Cottonelle | 13.9 |
| 10% NaCl/ Cottonelle | 17.5 |
| 15% NaCl/ Cottonelle | 23.9 |
| 20% NaCl/ Cottonelle | 41.7 |
| 23% NaCl/ Cottonelle | 56.2 |
| 5% NaCl/ Tucks | 16.2 |
| 10% NaCl/ Tucks | 17.9 |
| 15% NaCl/ Tucks | 24.7 |
| 20% NaCl/ Tucks | 39.9 |
| 23% NaCl/ Tucks | 51 |
| 36% Potassium Bromide | 16.4 |
| 43% Ammonium Sulfate | 105.9 |
| 41% Ammonium Nitrate | 14.7 |
| 4% Cetyltrimethyl Ammonium Bromide | 4.6 |
| 25% Ammonium Chloride | 15.3 |
| 5% Pentaerythritol | 1.5 |
| 33% Magnesium Sulfate | 40.5 |

### Example 2

This example in Table 2 shows the wet tensile data for flushable wipes prepared from a nonwoven substrate which comprised 75 grams/m² web.

**Table 2**

| **Flushable Wipes Solution** | **Avg. Peak Load (g/5cm)** |
|---|---|
| Dry | 691.7 |
| H2O | 18.5 |
| 4% Boric Acid | 79.9 |
| 25% Sodium Chloride | 160.8 |
| 25% Sodium Chloride | 155.6* |
| 1% Aerosol OT | 16 |
| Disponil FES 32 | 84.4 |
| SLS | 51.7 |
| Polystep B-27 | 294.2 |
| Aerosol A-102 | 74.7 |
| 5% Sodium Bicarbonate | 29.6 |
| 25% Sodium Sulfate | 263.2 |
| 6% Disodium Phosphate | 32.2 |
| 37% Sodium Citrate | 208.5 |
| 50% Ammonium Acetate | 397.8 |

| | |
|---|---|
| * 30 day soak | |

- Aerosol OT - sodium dioctyl sulfosuccinate from Cytec Industries Inc.
- Disponil FES 32 -- sodium laureth sulfonate (4 moles ethylene oxide; 30% aq soln.) from Cognis.
- SLS ― sodium lauryl sulfate
- Polystep B-27 ― sodium nonylphenol ethoxylate sulfate (3 moles ethylene oxide; 30% aq soln.) from Stepan Company.
- Aerosol A-102 ― disodium laureth sulfosuccinate (6 moles ethylene oxide; 30% aq soln.) from Cytec Industries Inc.

All the webs of Tables 1 and 2 disintegrated within 15 seconds of being tossed into agitated tap water at room temperature according to the Snag Breakup test.

It can be seen from the data in Tables 1 and 2 that with an appropriate minimum loading of a water-binding compound an aqueous lotion composition can be obtained that will provide a suitable wet tensile strength for the nonwoven web and yet allow the web to be disposable and disintegrate in an environment containing sufficient water to dissolve/redisperse the water soluble/dispersible component of the binder composition.

### STATEMENT OF INDUSTRIAL APPLICATION

The present invention provides a pre-moistened, disposable wet wipe which exhibits acceptable wet tensile strength but rapid disintegration in water.

## Claims

1. A packaged pre-moistened wet wipe comprising a web of nonwoven fibers bonded with a water soluble and/or dispersible polymeric binder in contact with an aqueous composition which is substantially free of boric acid and its derivatives and contains a sufficient amount of a water-binding compound to prevent the dissolution/redispersion of the water soluble/dispersible components of the binder composition while imbuing the nonwoven with a wet tensile strength that is at least as great as that afforded by a 4 wt% boric acid solution.

2. The wet wipe of Claim 1 in which the aqueous composition comprises 40-99 wt% water.

3. The wet wipe of Claim 1 in which the aqueous composition comprises less than 0.05 wt% boric acid or its derivatives.

4. The wet wipe of Claim 1 in which the water-binding compound is an ammonium, monovalent metal or polyvalent metal salt of an inorganic or organic acid.

5. The wet wipe of Claim 1 in which the water-binding compound is an ammonium, alkali metal or alkaline earth metal salt of carbonic acid, citric acid, acetic acid, succinic acid, phosphoric acid, sulfuric acid or hydrochloric acid.

6. The wet wipe of Claim 1 in which the water-binding compound is an ammonium, sodium or potassium salt of sulfuric acid.

7. The wet wipe of Claim 1 in which the water-binding compound is a humectant or a preservative.

8. The wet wipe of Claim 1 in which the water-binding compound is disodium cocoamphodiactetate or potassium sorbate.

9. The wet wipe of Claim 1 in which the polymeric binder comprises a polyvinyl alcohol, an aqueous polymer emulsion either stabilized with polyvinyl alcohol as the protective colloid, or an aqueous polymer emulsion containing post-added polyvinyl alcohol, a polyethylene glycol, a polyacrylamide or a polyvinyl pyrrolidone.

10. The wet wipe of Claim 1 in which the polymeric binder comprises a polyvinyl alcohol, an aqueous polymer emulsion either stabilized with polyvinyl alcohol as the protective colloid, an aqueous polymer emulsion containing post-added polyvinyl alcohol, or a combination thereof.

11. In a packaged pre-moistened wet wipe comprising a web of nonwoven fibers bonded with a polyvinyl alcohol-containing polymeric binder in contact with an aqueous lotion composition comprising in water one or more of an alcohol, a preservative, a cleansing agent, a fragrance, a moisturizer, a humectant, a surfactant and a softener, the improvement which comprises an aqueous lotion composition which contains less than 0.05 wt% boric acid and its derivatives and contains 3 to about 100 wt%, based on lotion solids, of a water-binding compound to prevent the dissoiution/redispersion of the polyvinyl alcohol-containing polymeric binder while imbuing the nonwoven with a wet tensile strength that is at least as great as that afforded by a 4 wt% boric acid solution.

12. The wet wipe of Claim 11 in which the polymeric binder comprises a polyvinyl alcohol, an aqueous polymer emulsion either stabilized with polyvinyl alcohol as the protective colloid, an aqueous polymer emulsion containing post-added polyvinyl alcohol, or a combination thereof.

13. The wet wipe of Claim 12 in which the water-binding compound is an ammonium, monovalent metal or polyvalent metal salt of an inorganic or organic acid.

14. The wet wipe of Claim 12 in which the water-binding compound is an ammonium, alkali metal or alkaline earth metal salt of carbonic acid, citric acid, acetic acid, succinic acid, phosphoric acid, sulfuric acid or hydrochloric acid.

15. The wet wipe of Claim 12 in which the water-binding compound is an ammonium, sodium or potassium salt of sulfuric acid.

16. The wet wipe of Claim 12 in which the water-binding compound is sodium sulfate.

17. The wet wipe of Claim 12 in which the water-binding compound comprises 5-95 wt% of the lotion solids.

18. The wet wipe of Claim 13 in which the water-binding compound comprises 5-95 wt% of the lotion solids.

19. The wet wipe of Claim 15 in which the water-binding compound comprises 5-95 wt% of the lotion solids.

20. The wet wipe of Claim 16 in which the water-binding compound comprises 5-95 wt% of the lotion solids.

21. In an aqueous lotion solution for wetting a nonwoven wipe bonded with a polyvinyl alcohol-containing polymeric binder, the improvement which comprises an aqueous lotion composition which is substantially free of boric acid and contains a sufficient amount of a water-binding compound to prevent the dissolution/redispersion of the polyvinyl alcohol-containing polymeric binder while imbuing the nonwoven with a wet tensile strength that is at least as great as that afforded by a 4 wt% boric acid solution.
